# EUROPÄISCHE PATENTANMELDUNG

(11) **EP 3 025 644 A1**
(43) Veröffentlichungstag der Anmeldung: **01.06.2016**
(21) Anmeldenummer: 15190506.4
(22) Anmeldetag: 20.10.2015
(51) Int. Cl.: A61B 5/0408

(54) **SENSORANORDNUNG ZUR ABLEITUNG ELEKTRISCHER SIGNALE**

(30) Priorität: 27.11.2014 US 201462085248 P
(71) Anmelder: BIOTRONIK SE & Co. KG, 12359 Berlin (DE)
(72) Erfinder: Bartels, Marc, 10115 Berlin (DE); Philipp, Jens, verstorben (DE); Fandrey, Stephan, 8910 Affoltern am Albis (CH)
(74) Vertreter: Galander, Marcus

(57) **Zusammenfassung**

Die Erfindung betrifft eine Sensoranordnung, die einen Primärsensor in Form einer selbstklebenden Oberflächenelektrode zur Ableitung elektrischer Signale aufweist, die eine zur Kontaktierung von Haut vorgesehene Sensorseite hat, sowie einen von dem Primärsensor verschiedenen Sekundärsensor zum Erfassen einer weiteren physikalischen Größe. Außerdem weist die Sensoranordnung einen Adapter auf, über den der Primärsensor und der Sekundärsensor zu einer lösbaren Einheit miteinander verbunden sind.

## Beschreibung

Die Erfindung betrifft eine Sensoranordnung mit einem Primärsensor in Form einer selbstklebenden Oberflächenelektrode zur Ableitung elektrischer Signale.

Selbstklebende Oberflächenelektroden zur Ableitung elektrischer Signale sind grundsätzlich bekannt und haben eine zur Kontaktierung von Haut vorgesehene Sensorseite, die selbstklebend ist, um die Oberflächenelektrode auf Haut aufkleben zu können und so einen sicheren Kontakt zur Haut herzustellen.

Selbstklebende Oberflächenelektroden zur Ableitung elektrischer Signale bei Mensch und Tier werden seit langem routinemäßig in Klinik und Forschung eingesetzt.

Erstellt werden mit diesen Elektroden beispielsweise Elektrokardiogramme (EKG) für die Herzaktivität, Elektromyogramme (EMG) für Muskelaktivitäten, Elektroneurogramme (ENG) für die Reizleitungsgeschwindigkeit bei Nerven, Elektrookulogramme (EOG) für die Augenbewegung.

Viele dieser Elektroden werden mit einer separaten oder bereits in die Elektrode integrierten Klebefolie auf der Haut des Probanden befestigt und dann über einen Stecker mit der Auswerteeinheit verkabelt.

Bei der Ableitung von Elektroenzephalogrammen (EEG) für die Hirnaktivität werden sowohl beidseitige Klebefolien, als auch Hauben verwendet, an denen die Elektroden befestigt werden.

Bei EKGs sind diese Elektroden typischerweise Einwegartikel.

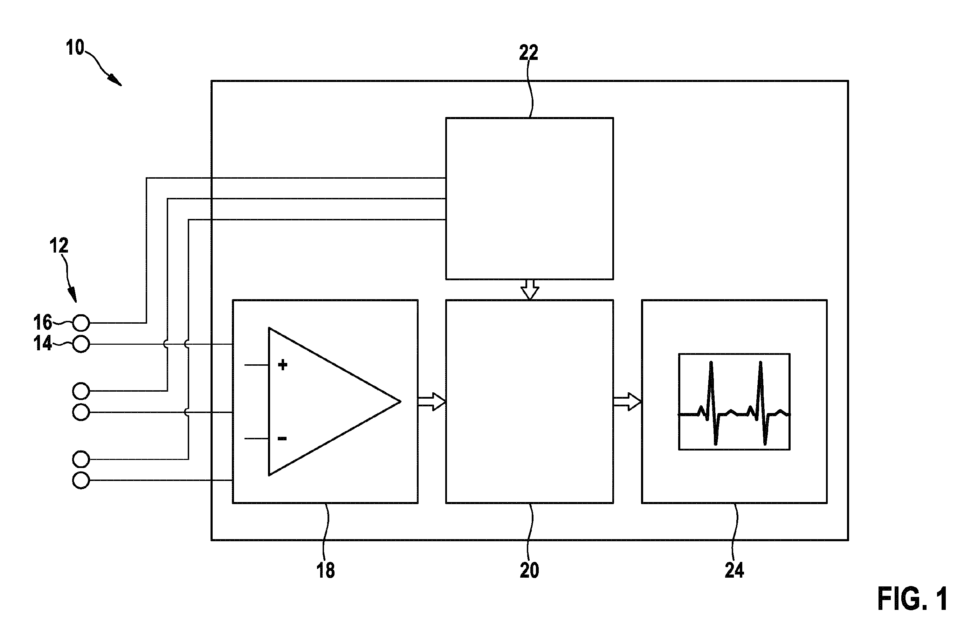

Die eigentliche aktive Fläche der EKG-Elektrode ist häufig rund und, mit oder ohne Kontaktgel-Reservoir, von einer selbstklebenden Kunststofffolie umgeben. Die Kontur dieser Folie variiert je nach Typ von einer runden Bauform, einer quadratischen, bis hin zu einer komplexen, mit Aussparungen versehenen Geometrie, s.Abb.1.a)-c). Die eigentliche Elektrodenfläche ist typischerweise rund und hat einen Durchmesser von ca. 5-8 mm.

Bei EKGs werden typischerweise drei bis zwölf Elektroden um das Herz und an den Extremitäten definiert positioniert.

Aufgabe der Erfindung ist es, den Anwendungsbereich selbstklebender Oberflächenelektroden zu erweitern oder die Anwendung selbstklebender Oberflächenelektroden zu vereinfachen.

Erfindungsgemäß wird diese Aufgabe durch eine Sensoranordnung gelöst, die einen Primärsensor in Form einer selbstklebenden Oberflächenelektrode zur Ableitung elektrischer Signale aufweist, die eine zur Kontaktierung von Haut vorgesehene Sensorseite hat, sowie einen von dem Primärsensor verschiedenen Sekundärsensor zum Erfassen einer weiteren physikalischen Größe. Außerdem weist die Sensoranordnung einen Adapter auf, über den der Primärsensor und der Sekundärsensor zu einer lösbaren Einheit miteinander verbunden sind.

Dies ermöglicht eine einfache und zuverlässige Anwendung selbstklebender Oberflächenelektroden mit jeweils einem von diesem verschiedenen Sekundärsensor zum Erfassen einer weiteren physikalischen Größe. Mit Hilfe des mechanischen Adapters, können handelsübliche (Selbstklebe-) Ableitungselektroden in Kombination mit weiteren im Körperkontakt befindlichen Sensoren verwendet werden.

Ein Anwendungsgebiet, in dem dies besonders vorteilhaft ist, ist die Ableitung von Elektrokardiogrammen an Patienten in einem Magnetresonanztomographen.

Neben der Fluoroskopie ist das n-Kanal-EKG (n>1) das wichtigste Instrument in einer Elektrophysiologischen Untersuchung (EPU), um kardiologische Arrhythmien zu diagnostizieren, beispielsweise bei Katheterisierungen und Ablationen.

Die Magnetresonanztomographie (MRT) bietet wie kaum eine andere nicht-ionisierende Modalität räumlich und zeitlich aufgelösten Weichteilgewebekontrast und ermöglicht dadurch ein breites Spektrum an diagnostischen Anwendungen für schwer zugängliche Organe bzw. Anatomien wie dem Gehirn, der Wirbelsäule, den Gelenken, den Abdomen, dem kardiovaskulärem System etc.

Eine Kombination beider medizinischer Messverfahren würde es ermöglichen, eine EPU im MRT durchzuführen hin zu interventionellem bzw. intraoperativem MR (iMR) mit dem Ziel, Ablationen präziser und vor allem mit der Unterstützung von Bildgebungsverfahren ohne ionisierende Strahlen zum Schutze von Patient und Krankenhauspersonal durchzuführen.

Das direkte Zusammenführen von EKG und MRT bereitet aus physikalischen Gründen technische Herausforderungen, insbesondere durch die drei von einander unabhängigen und demzufolge getrennt adressierbaren Hauptkomponenten des MRT, dem starken homogenen statischen Magnetfeld im Tesla-Bereich (1.), den drei orthogonal zueinander gerichteten magnetischen Gradienten- (2.) und den elektromagnetischen Hochfrequenzfeldern(3.). Die Erfindung adressiert ausschließlich das störungsfreie Messen von elektrophysiologischen Signalen im Umfeld von starken homogenen statischen Magnetfeldern (1), die durch den Magnetohydrodynamischen Effekt (MHD) verfälscht werden. Störungen durch magnetische Gradienten- und elektromagnetische Hochfrequenzfelder werden in dieser Offenbarung nicht gelöst, da es dafür unabhängige Lösungsstrategien gibt.

Der magnetohydrodynamische Effekt (MHD) hat seine physikalische Ursache in bewegten Ladungsträgern in einem Magnetfeld, die durch die Lorentzkraft in eine bestimmte Richtung gezwungen werden, abhängig von der vektoriellen Bewegungsrichtung der Ladungsträger und der Ausrichtung des Magnetfeldes. Der MHD als pseudo-periodische elektrophysiologische Störgröße im EKG wird hauptsächlich durch den Blutfluss im Aortenbogen während der Systole induziert. Dabei werden bewegte Ladungsträger im Blut durch die Lorentzkraft an die Blutgefäßwand gedrückt und temporär dort lokal akkumuliert. Diese temporäre Ladungsdichte überlagert sich als Potential über das aufzunehmende EKG, insbesondere in dessen QRS-Komplex und kann mit dem EKG auf der Hautoberfläche des Patienten gemessen werden.

Die vorgeschlagenen Korrekturverfahren erfassen eine zusätzliche Messgröße, die die Herzaktivität charakterisiert, aber vom MHD nicht beeinflusst wird, z.B. über akustische oder optische Sekundärsensoren, deren Signale mit denen des Primärsensors (EKG-Elektrode) geeignet verrechnet werden.

Die erfindungsgemäße Sensoranordnung bietet den Vorteil, das die jeweiligen Sekundärsensoren nahe an der jeweiligen EKG-Elektrode (also der Oberflächenelektrode) am Körper befestigt werden können, so dass in optimaler Weise jeder EKG-Kanal getrennt korrigiert werden kann.

Die erfindungsgemäße Sensoranordnung bietet eine stabile, reproduzierbare Anordnung des Primär- und des Sekundärsensors sowohl zueinander als auch auf der Hautoberfläche des Probanden. Der klinische Ablauf ist durch die Einführung des zusätzlichen Sekundärsensors kaum beeinflusst. Außerdem erlaubt es, die erfindungsgemäße Sensoranordnung Sekundärsensoren einzusetzen, die einen direkten Körperkontakt benötigen, wie dies bei akustischen und optischen Sensoren der Fall ist. Außerdem können mit der erfindungsgemäßen Sensoranordnung beliebige EKG-Elektroden verwendet werden, insbesondere auch Einwegelektroden.

Die Erfinder haben erkannt, dass es nachteilig ist, den Primär- und den Sekundärsensor zu einer festverbundenen Einheit zu kombinieren. Nachteile einer solchen Lösung sind
- Da der Sekundärsensor möglicherweise aus Kostengründen wiederverwendbar sein sollte, lässt sich kein Einwegprodukt gestalten.
- Der Untersucher kann seine favorisierte Primärelektrode nicht mehr verwenden.

Diese Lösung funktioniert allerdings nicht, wenn der Sekundärsensor einen direkten Körperkontakt benötigt, wie dies bei akustischen und optischen Sensoren der Fall ist.

Bei der erfindungsgemäßen Sensoranordnung wird der Sekundärsensor auf einem geeignet ausgeformten Adapter angebracht oder fest integriert. Der Adapter ist so ausgeformt, dass er mit an sich bekannten, selbstklebenden Oberflächenelektroden zur Ableitung elektrischer Signale kombiniert werden kann.

Vorzugsweise sind der Adapter und der Sekundärsensor fest miteinander verbunden, während der Primärsensor lösbar mit dem Adapter verbunden ist.

Der Adapter ist vorzugsweise ausgebildet, an einer von der zur Kontaktierung mit Haut vorgesehenen Sensorseite des Primärsensors abgewandten Sensorseite des Primärsensors an dieser lösbar befestigt zu werden.

In einer besonders bevorzugten Ausführungsvariante weist der Adapter einen flachen Klebeabschnitt auf, der zur unmittelbaren Befestigung an Haut ausgebildet ist und eine zur Kontaktierung von Haut vorgesehene Adapterseite aufweist. Der Klebeabschnitt hat eine Aussparung, die so angeordnet und ausgebildet ist, dass ein an der der zur Kontaktierung von Haut vorgesehenen Adapterseite abgewandten Adapterseite aufgebrachter Primärsensor durch die Aussparung hindurch unmittelbaren Hautkontakt haben kann.

Vorzugsweise ist die Aussparung wenigstens annähernd zentral in dem Klebeabschnitt angeordnet.

Der Klebeabschnitt kann um die Aussparung herum mehrere, zur Aussparung konzentrische Markierungen aufweisen, die es erleichtern, den Primärsensor zentriert zur Aussparung außen auf dem Klebeabschnitt des Adapters anzubringen, so dass der Primärsensor durch die Aussparung hindurch guten Hautkontakt hat.

Vorzugsweise weist der Adapter einen über den übrigen Klebeabschnitt seitlich hinausragenden Abschnitt auf, der den Sekundärsensor und/oder einen Steckverbinder für den Sekundärsensor trägt.

Der Sekundärsensor ist vorzugsweise nicht zentral, sondern an einem oder in der Nähe eines Randes - also peripher - an dem Adapter befestigt, so das der Primärsensor zentral auf dem Adapter befestigt werden kann.

Die Sensoranordnung weist vorzugsweise einen Steckverbinder für den Sekundärsensor auf, der mit dem Sekundärsensor elektrisch verbunden ist und der an dem Adapter befestigt ist. Über den Steckverbinder kann auch der Sekundärsensor mit einem Gerät zur Verarbeitung der vom Sekundärsensor erfassten Signale mittels Kabel verbunden werden. Alternativ könnte der Sekundärsensor auch mittel zur drahtlosen Signalübertragung aufweisen.

Ein Adapter mit Sekundärsensor für eine Sensoranordnung - also ohne befestigten Primärsensor - stellt einen selbstständig schutzfähigen Gegenstand dar.

Vorzugsweise ist der Sekundärsensor ein akustischer Sensor und/oder ein Beschleunigungssensor und/oder ein optischer Sensor. Derartige Sensoren können so ausgeführt sein, dass sie durch einen Magnetresonanztomographen gar nicht oder nur wenig beeinflusst werden. Auch Ultraschallsensoren sind daher geeignete Sekundärsensoren.

Vorzugsweise besteht der Adapter wenigstens teilweise aus einem weichen, biokompatiblen Kunststoff, z.B. Silikon, beschichtetem oder unbeschichtetem Papier, Gewebe aus Natur- oder Kunststoffen.

Die Erfindung soll nun anhand von Ausführungsbeispielen mit Bezug auf die Figuren näher erläutert werden. Von den Figuren zeigt:
- Figur 1:: Ein Elektrokardiographiesystem mit einer erfindungsgemäßen Sensoranordnung;
- Figuren 2a bis 2c:: verschiedene typische Ausführungsformen von herkömmlichen selbstklebenden Oberflächenelektroden für die Ableitung elektrischer Signale;
- Figur 3:: eine erste Variante einer erfindungsgemäßen Sensoranordnung mit einem Adapter, der über einen Primärsensor gestülpt wird;
- Figuren 4a bis 4c:: verschiedene zweite Varianten, bei denen der Primärsensor auf den Adapter aufgesetzt wird;
- Figuren 5a bis 5c:: verschiedene Varianten der Kontaktierung des Sekundärsensors auf dem Adapter; und
- Figur 6:: einen Adapter mit konzentrischen Kreisen als Zentrierhilfe für punktsymmetrische Primärsensoren.

Das in Figur 1 dargestellte Elektrokardiographiesystem 10 weist mehrere Sensoranordnungen 12 gemäß der Erfindung auf. Jede der Sensoranordnungen 12 weist einen Primärsensor 14 und einen Sekundärsensor 16 auf. Die Sekundärsensoren 16 sind herkömmliche selbstklebende Oberflächenelektroden für die Ableitung elektrischer Signale, beispielsweise für die Ableitung von Elektrokardiogrammen. Die Primärsensoren sind mit einer Signalaufbereitungseinheit 18 zum Filtern und Verstärken der abgeleiteten elektrischen Signale verbunden. Die so aufbereiteten elektrischen Signale werden einer Signalauswerteeinheit 20 zugeführt, die die abgeleiteten elektrischen Signale unter Berücksichtigung von Sekundärsignalen auswertet.

Zum Aufnehmen von Sekundärsignalen dienen die Sekundärsensoren 16, die mit einer Sekundärsignal-Aufbereitungseinheit 22 verbunden sind. Als Sekundärsensoren kommen beispielsweise optische oder akustische Sensoren oder auch Bewegungssensoren in Frage, die durch den Einsatz in einen Magnetresonanztomographen nicht oder nur wenig beeinflusst werden. Die Sekundärsensoren liefern im Ergebnis (nach Aufbereitung durch die Sekundärsensor-Aufbereitungseinheit 22) ein Sekundärsignal, auf Basis dessen die Signalauswerteeinheit 18 den Einfluss beispielsweise magnetohydrodynamischen Effekts auf abgeleitete EKG-Signale bestimmen und kompensieren kann. Das auf diese Weise bereinigte Elektrogramm kann dann über einen Drucker oder eine Anzeige 24 beispielsweise einem Arzt dargestellt werden.

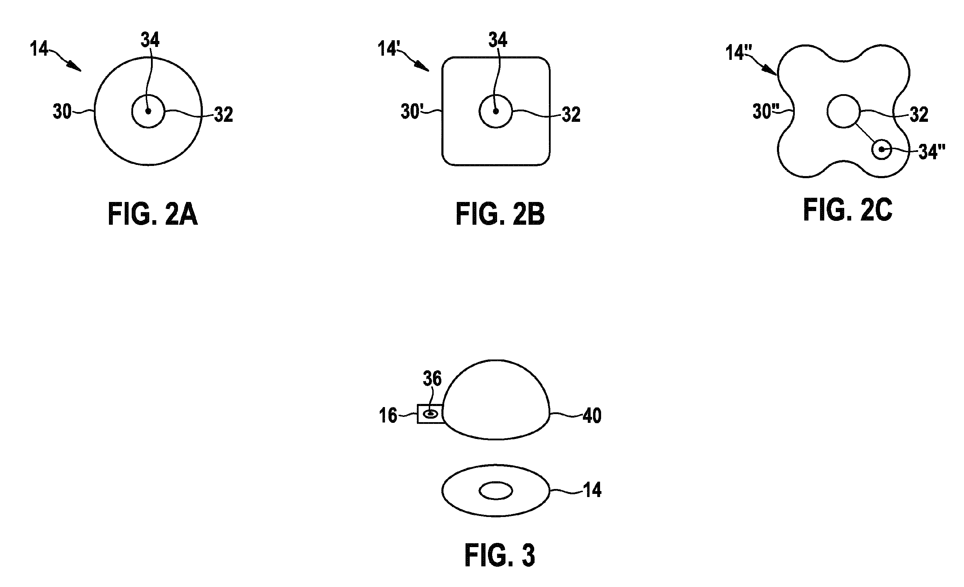

In Figur 1 sind die Sensoranordnungen 12 nur sehr schematisch dargestellt. Vorgeschlagene konkrete Ausgestaltungen sind in den Figuren 3 bis 6 dargestellt.

Figur 2 zeigt typische Ausführungsformen üblicher selbstklebender Oberflächenelektroden.

Figur 2a zeigt eine runde Oberflächenelektrode. Diese weist einen Träger 30 mit untenliegender Klebefläche auf. Innerhalb der Klebefläche ist eine Elektrodenfläche 32 vorgesehen, der gegebenenfalls ein ebenfalls unten angebrachtes Kontaktgelreservoir benachbart ist. Schließlich weist die Oberflächenelektrode 14 einen Steckverbinder 34 auf, der beispielsweise ein Druckknopf sein kann und dazu dient, die Elektrodenfläche 32 über ein Kabel elektrisch mit einer Signalaufbereitungseinheit eines Elektrokardiographen zu verbinden.

Die in den Figuren 2a bis 2c dargestellten Ausführungsformen der Oberflächenelektrode 14, 14' und 14" unterscheiden sich durch die Form des jeweiligen Trägers 30, 30' bzw. 30". Gemäß der Ausführungsform in Figur 2a ist der Träger rund, während der gemäß Figur 2b eine eckige Bauform mit abgerundeten Ecken hat. In Figur 2c ist schließlich ein Träger 30" dargestellt, dessen Kontur Aussparungen für benachbarte Elektroden besitzt und auf diese Weise eine große Klebefläche realisieren kann. Der Steckverbinder 34" ist seitlich abgesetzt.

Figur 3 zeigt eine erste Variante einer erfindungsgemäßen Sensoranordnung mit einem Adapter 40, der auf einen Primärsensor, beispielsweise eine Oberflächenelektrode 14, aufgesetzt werden kann. In den Adapter 40 integriert ist ein Sekundärsensor 16 mit einem Steckverbinder 36. Der Adapter 40 ist so ausgebildet, dass er über den Primärsensor 14 gestülpt werden kann oder huckepack auf diesen aufgesetzt werden kann und zum Beispiel durch integrierte Klebeflächen auf dem Primärsensor 14 gehalten wird.

Der Adapter 40 hat eine Grundfläche, die an mindestens dem Ort, an dem der Sekundärsensor 16 am Adapter befestigt ist, größer ist als die Grundfläche (d.h. der Träger 30) der Primärelektrode. Auf diese Weise hat auch der Sekundärsensor 16 unmittelbaren Hautkontakt. Ein Nachteil dieser ersten Variante ist, dass die verschiedenen Oberflächenelektroden 14 sehr unterschiedliche Bauformen und Abmessungen haben können, so dass möglicherweise entsprechend unterschiedliche Adapter 40 vorgesehen werden müssen.

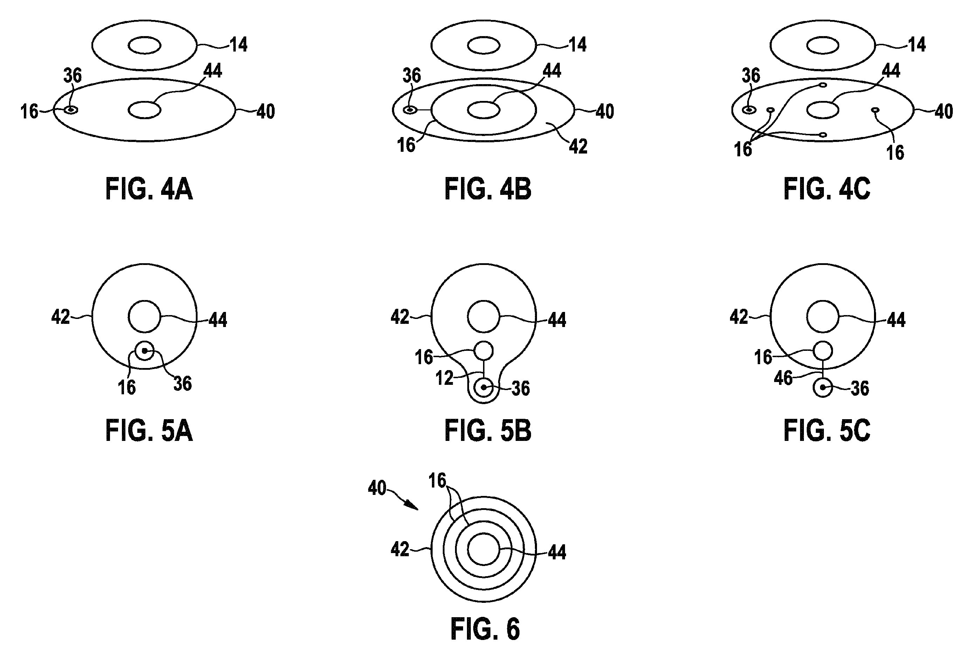

Figur 4 zeigt schließlich eine zweite Variante einer erfindungsgemäßen Sensoranordnung, bei der ein jeweiliger Primärsensor auf den Adapter aufgesetzt wird. Auch bei dieser Variante bilden der Adapter 40' und der jeweilige Sekundärsensor 16 eine Einheit. Der Adapter 40' hat eine zur Befestigung auf Haut vorgesehene Klebefläche (in der Abbildung auf der Unterseite), die sich auf der Unterseite eines Klebeabschnitts 42 (Träger des Adapters) befindet. Mit dem Klebeabschnitt 42 ist der Sekundärsensor 16 fest verbunden.

Der Klebeabschnitt 42 besitzt eine Aussparung 44, durch die ein außen auf den Klebeabschnitt 42 aufgebrachter Primärsensor unmittelbaren Hautkontakt haben kann. Dazu kann die Außenseite (also die bei Benutzung der Haut abgewandte Seite) des Klebeabschnitts 42 des Adapters 40 so ausgebildet sein, dass sich auf ihr herkömmliche selbstklebende Oberflächenelektroden befestigen lassen. Außerdem ist der Klebeabschnitt 42 so dünn ausgebildet, dass der auf seiner Außenseite aufgebrachte Primärsensor keinen nennenswerten Abstand zur Haut aufweist.

Die in den Figuren 4a bis 4c dargestellten Untervarianten der zweiten Ausführungsvariante der erfindungsgemäßen Sensoranordnung unterscheiden sich durch die unterschiedliche Anordnung des jeweiligen Sekundärsensors 16 mit zugehörigem Steckverbinder 36. Gemäß der Variante in Figur 4a ist der Sekundärsensor 16 mit radialem Versatz zur Aussparung 44 und damit zum Primärsensor 14 angeordnet. Gemäß der Ausführungsvariante in Figur 4b ist der Sekundärsensor 16 konzentrisch zur Aussparung 44 angeordnet, wobei der Sekundärsensor-Steckverbinder 36 seitlich versetzt angeordnet ist, um den Primärsensor 14 ebenfalls zentrisch zur Aussparung 44 anbringen zu können.

Gemäß Figur 4c wird der Sekundärsensor 16 von einem Sekundärsensor-Array gebildet. Dieses Sekundärsensor-Array ist gleichmäßig um die Aussparung 44 herum angeordnet und besitzt ebenfalls einen Sekundärsensor-Steckverbinder 36, der seitlich versetzt am Rande des Klebeabschnitts 42 des Adapters 40 angeordnet ist.

Figuren 5a bis 5c zeigen schließlich verschiedene Varianten der Kontaktierung, d.h. der Anordnung des Steckverbinders 36 für den Sekundärsensor 16 auf dem Adapter 40.

Gemäß Figur 4a befindet sich der Steckverbinder 36 für den Sekundärsensor 16 unmittelbar über dem Sekundärsensor 16.

Gemäß der Ausführungsvariante in Figur 4b ist der Steckverbinder 36 für den Sekundärsensor seitlich gegenüber dem Sekundärsensor 16 versetzt auf einem seitlichen Vorsprung des Klebeabschnitts 42 vorgesehen.

Gemäß der Ausführungsvariante in Figur 5c ist schließlich der Steckverbinder 36 für den Sekundärsensor gar nicht auf dem Klebeabschnitt 42 angeordnet, sondern über ein Kabel 46 mit dem Primärsensor 16 verbunden.

Figur 6 zeigt schließlich, dass der Klebeabschnitt 42 des Adapters 40 Markierungen 48 in Form konzentrischer Ringe um die zentrale Aussparung 44 herum aufweisen kann. Diese Markierungen 48 dienen als Zentrierhilfe für das Aufbringen selbstklebender Oberflächenelektroden als Primärsensoren.

Der Primärsensor (z.B. EKG-Elektrode) wird auf dem Adapter befestigt, z.B. durch integrierte Klebeflächen. Der Adapter hat ein integriertes "Sichtfenster" durch das die Elektrode des Primärsensors sicheren Kontakt mit der Haut herstellen kann.

Der Adapter besitzt auf seiner Unterseite eine Klebefläche, mit der die Kombination auf der Haut befestigt werden kann.

Vorteilhaft bei dieser Lösung ist, dass die Primärelektrode eine beliebige Kontur haben kann. Somit kann der Untersucher seine bevorzugte EKG-Elektrode weiter verwenden.

Nachfolgend ist ein beispielhafter Ablauf der Anwendung bei Variante 2 skizziert:
- Bereitstellen der (steril verpackten) Primärelektrode (z.B. für EKG)
   - Bereitstellen des desinfizierten/sterilen Adapters mit der Sekundärelektrode
   - Befestigen der Adapter am Körper des Probanden. Anzahl der Adapter = Anzahl der EKG-Ableitungskanäle; Orte der Anbringung so, dass die Aussparungen in den Adaptern an den gewünschten Orten der EKG-Ableitungen liegen
   - Verkabelung der in den Adaptern befindlichen Sekundärsensoren mit ihrer Auswerteeinheit
   - Befestigung der Primärsensoren (EKG-Elektroden) auf den platzierten Adaptern
   - Verkabelung der Primärsensoren mit ihrer Auswerteeinheit
   - Untersuchung des Probanden
   - Entfernen der Verkabelung
   - Entsorgen der Einweg-EKG-Elektroden
   - Bereitstellen der Adapter / Sekundärelektroden für die Vorbereitung zum nächsten Einsatz, z.B. Entfernung der Klebefolie, Desinfektion

Die erfindungsgemäße Sensoranordnung bietet folgende Vorteile:
- Der klinische Ablauf wird möglichst wenig verändert
- Beliebige EKG-Elektroden können verwendet werden
- Der Einsatz von Einwegartikeln ist weiterhin möglich
- Die Variante 2 ist eine "One Size Fits All" Lösung

Der Einsatz von Sekundärsensoren wird durch den erfindungsgemäßen Adapter vereinfacht. Dadurch werden Systeme einfacher bedienbar, die einen solchen Sekundärsensor benötigen. Ein solches System könnte z.B. ein MRT-kompatibles EKG-Aufzeichnungsgerät sein, aber auch ein MRT-kompatibles System für Elektrophysiologische Untersuchungen.

### Bezugszeichenliste

- 10: Elektrokardiographiesystem
- 12: Sensoranordnung
- 14: Primärsensor
- 16: Sekundärsensor
- 18: Signalaufbereitungseinheit
- 20: Signalauswerteeinheit
- 22: Sekundärsignal-Aufbereitungseinheit
- 24: Anzeige-/Drucker
- 30: Träger
- 32: Elektrodenfläche
- 34: Steckverbinder des Primärsensors
- 36: Steckverbinder des Sekundärsensors
- 40: Adapter
- 42: Klebeabschnitt
- 44: Aussparung
- 46: Kabel
- 48: Markierungen

## Patentansprüche

1. Sensoranordnung (12) mit
- einem Primärsensor (14) in Form einer selbstklebenden oder mit Kleber zu versehenden oder anderem Befestigungsmaterial und/oder Befestigungsmechanismus ausgestatteten Oberflächenelektrode zur Ableitung elektrischer Signale, die eine zur Kontaktierung von Haut vorgesehene Sensorseite hat, sowie mit
- einem von dem Primärsensor (14) verschiedenen Sekundärsensor (16) zum Erfassen einer weiteren physikalischen Größe,
**gekennzeichnet durch**
- einen Adapter (40), über den der Primärsensor (14) und der Sekundärsensor (16) zu einer lösbaren Einheit miteinander verbunden sind.

2. Sensoranordnung (12) nach Anspruch 1, **dadurch gekennzeichnet, dass** der Adapter (40) und der Sekundärsensor (16) fest miteinander verbunden sind und der Primärsensor (14) lösbar mit dem Adapter (40) verbunden ist.

3. Sensoranordnung (12) nach Anspruch 1 oder 2, **dadurch gekennzeichnet, dass** der Adapter (40) ausgebildet ist, an einer von der zur Kontaktierung mit Haut vorgesehenen Sensorseite des Primärsensors (14) abgewandten Sensorseite des Primärsensors (14) an diesem lösbar befestigt zu werden.

4. Sensoranordnung (12) nach Anspruch 1 oder 2, **dadurch gekennzeichnet, dass** der Adapter (40) einen flachen Klebeabschnitt (42) aufweist, der zur unmittelbaren Befestigung an Haut ausgebildet ist und eine zur Kontaktierung von Haut vorgesehene Adapterseite aufweist, wobei der Klebeabschnitt (42) eine Aussparung (44) aufweist, die so angeordnet und ausgebildet ist, dass ein an der der zur Kontaktierung von Haut vorgesehenen Adapterseite abgewandten Adapterseite aufgebrachter Primärsensor durch die Aussparung (44) hindurch unmittelbaren Hautkontakt haben kann.

5. Sensoranordnung (12) nach Anspruch 4, **dadurch gekennzeichnet, dass** die Aussparung (44) wenigstens annähernd zentral in dem Klebeabschnitt (42) angeordnet ist.

6. Sensoranordnung (12) nach Anspruch 4 oder 5, **dadurch gekennzeichnet, dass** der Klebeabschnitt (42) um die Aussparung (44) herum mehrere, zur Aussparung (44) konzentrische Markierungen (48) aufweist.

7. Sensoranordnung (12) nach wenigstens einem der Ansprüche 4 bis 6, **dadurch gekennzeichnet, dass** der Adapter (40) einen über den übrigen Klebeabschnitt seitlich hinausragen Abschnitt aufweist, der den Sekundärsensor (16) und/oder einen Steckverbinder (36) für den Sekundärsensor (16) trägt.

8. Sensoranordnung (12) nach wenigstens einem der Ansprüche 1 bis 7, **dadurch gekennzeichnet, dass** der Sekundärsensor (16) an einem oder in der Nähe eines Randes an dem Adapter (40) befestigt ist.

9. Sensoranordnung (12) nach wenigstens einem der Ansprüche 1 bis 8, **gekennzeichnet durch** einen Steckverbinder (36) für den Sekundärsensor (16), der mit dem Sekundärsensor elektrisch verbunden ist und der an dem Adapter (40) befestigt ist.

10. Adapter (40) mit Sekundärsensor (16) für eine Sensoranordnung (12) gemäß wenigstens einem der Ansprüche 1 bis 9.

11. Adapter (40) gemäß Anspruch 10, **dadurch gekennzeichnet, dass** er mit einem Sekundärsensor (16) in Form eines akustischen Sensors und/oder eines Beschleunigungssensors und/oder eines optischen Sensors versehen ist.

12. Adapter (40) gemäß Anspruch 10 oder 11, **dadurch gekennzeichnet, dass** er mit einem Sekundärsensor (16) in Form eines Ultraschallsensors versehen ist.

13. Adapter (40) gemäß wenigstens einem der Ansprüche 10 bis 12, **dadurch gekennzeichnet, dass** der Adapter (40) wenigstens teilweise aus einem weichen, biokompatiblen Kunststoff besteht.
